# EUROPÄISCHE PATENTSCHRIFT

(11) **EP 1 047 374 B1**
(45) Veröffentlichungstag und Bekanntmachung des Hinweises auf die Patenterteilung: **03.01.2007**
(21) Anmeldenummer: 99958021.0
(22) Anmeldetag: 11.11.1999
(51) Int. Cl.: A61K 8/67, A61Q 5/04

(54) **MITTEL UND VERFAHREN ZUR DAUERHAFTEN HAARVERFORMUNG**
COMPOSITION AND PROCESS FOR PERMANENT HAIRSHAPING
AGENT ET METHODE POUR PERMANENTES

(30) Priorität: 14.11.1998 DE 19852611
(43) Veröffentlichungstag der Anmeldung: 02.11.2000
(73) Patentinhaber: Wella Aktiengesellschaft, 64274 Darmstadt (DE)
(72) Erfinder: DANNECKER, Beate, D-64283 Darmstadt (DE); LAUSCHER, Dirk, D-64372 Ober-Ramstadt (DE); SCHREIBER, Birgit, D-64678 Lindenfels (DE)
(86) Internationale Anmeldenummer: PCT/EP1999/008669
(87) Internationale Veröffentlichungsnummer: WO 2000/028951

(56) Entgegenhaltungen:
- FR-A- 2 176 697
- US-A- 2 780 579
- US-A- 5 051 252

## Beschreibung

Die vorliegende Erfindung betrifft ein Mittel in Form von mehreren Komponenten in dem Dehydroascorbinsäure, ihrer Derivate oder Salze wasserfrei, vorzugsweise in fester Form, vorliegen zur Durchführung der oxidativen Nachbehandlung bei der reduktiven Veränderung von Keratinfasern, insbesondere der dauerhaften Haarverformung.

Bei der reduktiven Veränderung von Keratinfasern wird das Haar zunächst mit einem Verformungsmittel, welches eine Öffnung der Disulfidbindungen des Haarkeratins bewirkt, behandelt und sodann in die gewünschte Form gebracht. Als Verformungsmittel werden hierbei in der Regel keratinreduzierende Mercaptoverbindungen, wie zum Beispiel Salze oder Ester von Mercaptocarbonsäuren, verwendet. Anschließend wird das Haar mit Wasser oder einem geeigneten Zwischenbehandlungsmittel gespült. Sodann werden die reduzierten Haarfasern mit einem Fixiermittel oxidativ nachbehandelt. Hierbei werden Disulfidbindungen innerhalb des Haarkeratins geschlossen, welche für die dauerhafte Haltbarkeit der Haarverformung, insbesondere beim Wellen oder Glätten, maßgebend sind.

Bei Fixiermitteln auf der Basis von Wasserstoffperoxid, Peroxidsalzen oder Bromaten wird ein Teil der Disulfid- und Thiolgruppen des Haarkeratins zu höheren Oxidationsstufen des Schwefels, insbesondere zu Cysteinsäure aufoxidiert. Hierdurch wird das Haarkeratin irreversibel geschädigt. Im Falle peroxidhaltiger Fixiermittel wird außerdem das Farbpigment des Haares (Melanin) partiell zerstört, was mit einer Aufhellung des Haares verbunden ist.

Es sind verschiedene Fixiermittel auf Disulfidbasis bekannt, die diese Nachteile lösen sollen:

Aus der JP-OS 04-112 818 ist ein Fixiermittel für Dauerwellen auf der Basis von Riboflavin und Glutathion (als oxidierendes Agens) vorbekannt. In der EP-OS 0 448 185 wird ein Verfahren zur Behandlung von Keratinmaterial beansprucht, bei dem Disulfide der allgemeinen Formel Z-R-S-S-R-Z (Z = wasserlösliche Gruppe, insbesondere Amino-; R = zweiwertiger Rest mit mindestens 2 Kohlenstoffatomen, insbesondere C₂ bis C₁₀) zur Reoxidation der reduktiv erhaltenen Sulfhydrylgruppen verwendet werden. Bevorzugte Disulfide sind Cystamin und Glutathiondisulfid.

Disulfidhaltige Fixiermittel sind mit dem Nachteil behaftet, daß während des Fixiersschrittes Thiole entstehen, die einen unangenehmen Geruch aufweisen.

Die Verwendung von Dehydroascorbinsäure zur Einstellung eines sauren pH-Wertes bei Haarkonditionierungsmitteln ist aus der JP-OS 52-128 239 bekannt. Dehydroascorbinsäure ist in wäßriger Lösung instabil und zersetzt sich innerhalb weniger Stunden. Die US 2780579 betrifft Mittel und Verfahren zur dauerhaften Haarverformung unter Verwendung einer Fixierung mit einem Gehalt an Dehydroascorbinsäure. Die Dehydroascorbinsäure soll dort bei pH 2 bis 10, in einer Menge von 0,03 bis 0,8 Gew.% eingesetzt werden.

Es stellte sich die Aufgabe, die bei der dauerhaften Haarverformung auftretenden Nachteile bezüglich Bleichwirkung, Cysteinsäurebildung und Mercaptangeruch zu vermeiden, ohne hierdurch die Haarstruktur zu beeinträchtigen.

Überraschend wurde gefunden, daß die gestellte Aufgabe in hervorragender Weise durch ein Mittel für die Durchführung der oxidativen Nachbehandlung von zuvor reduktiv behandelten Haaren bei der dauerhaften Haarverformung gemäß Anspruch 1 gelöst wird.

Das Fixiermittel wird erhalten durch Vermischen von 2 Komponenten unmittelbar (10 sec bis 20 min) vor dem Gebrauch, wobei die Komponente 1 Dehydroascorbinsäure, ihrer Derivate oder Salze oder deren Gemisch, wasserfrei oder mit bis zu 10 Gewichtsprozent Wasser, als Pulver-, Granulat- oder Tablette oder mikroverkapselt oder als Suspension enthält und die Komponente 2 als wäßrige, alkoholische oder wäßrig-alkoholische Zubereitung vorliegt.

Für das erfindungsgemäße Mittel geeignet sind, neben Dehydroascorbinsäure, insbesondere ihre Derivate Dehydroisoascorbinsäure, bis-Dehydroascorbinsäure, bis-Dehydroisoascorbinsäure sowie deren Salze, wie zum Beispiel Alkali- und Erdalkalimetallsalze, sowie Gemischen daraus. Besonders bevorzugte sind Dehydroascorbinsäure oder bis-Dehydroascorbinsäure oder Mischungen daraus.

Besonders bevorzugt enthält das Fixiermittel, 1 bis 5 Gewichtsprozent Dehydroascorbinsäure oder bis-Dehydroascorbinsäure oder Mischungen daraus.

Gegebenenfalls kann das Fixiermittel zusätzlich übliche Oxidationsmittel wie z. B. Wasserstoffperoxid, Peroxidsalze oder Bromate enthalten, wobei diese, falls sie flüssig vorliegen, in der flüssigen Komponente enthalten sind.

Der pH-Wert des gebrauchsfertigen Fixiermittels liegt in einem Bereich von 1,5 bis 10, vorzugsweise 2 bis 8, besonders bevorzugt 2,5 bis 7,5. Die Einstellung des pH-Wertes erfolgt mit üblichen Alkalisierungsmitteln und puffernden Stoffen wie zum Beispiel Ammoniak, Alkalihydroxide, Alkalicarbonate, Alkalihydrogencarbonate, Citratpuffer, Phosphorsäure und deren Salze, Zitronensäure und deren Salze und insbesondere Ascorbinsäure und deren Salze.

Das gebrauchsfertige Fixiermittel kann sowohl in Form einer wäßrigen Lösung oder einer Emulsion als auch in verdickter Form auf wäßriger Basis, insbesondere als Creme, Gel oder Paste vorliegen. Vorzugsweise wird die Dehydroascorbinsäure, ihrer Derivate oder Salze oder deren Gemisch in wässriger oder in wäßrigalkoholischer Lösung verwendet.

Neben der Dehydroascorbinsäure, ihrem Derivate oder ihrem Salze oder deren Gemisch kann das gebrauchsfertige Fixiermittel noch Zusätze enthalten, wie sie in haarkosmetischen Zubereitungen (vergleiche K. Schrader, Grundlagen und Rezepturen der Kosmetika, 2. Auflage, Hüthig-Verlag, Heidelberg, 1989) üblich sind. Hierzu gehören Quell- und Penetrationsstoffe, wie zum Beispiel Harnstoff, 2-Pyrrolidon, 1-Methyl-2-pyrrolidon und Dipropylenglykolmonomethylether, sowie Peroxidstabilisatoren, beispielsweise aromatische Sulfonsäuren, Salzsäure, Schwefelsäure, Phosphorsäure, Pyro- oder Polyphosphorsäuren, saure Salze starker Säuren, Ascorbinsäure, Oxalsäure, Malonsäure, Benzoesäure, Salicylsäure, Zitronensäure, Gerbsäuren, Paraformaldehyd, 4-Acetamido-phenol, Phenol, Thymol oder alpha-Bisabolol, zugesetzt werden.

Weiterhin können in dem Fixiermittel Netzmittel und Emulgatoren aus der Gruppe der anionischen, nichtionischen, kationischen und amphoteren oder zwitterionischen oberflächenaktiven Agenzien enthalten sein. Geeignete oberflächenaktive Agenzien sind insbesondere
a) anionische oberflächenaktive Agenzien, wie beispielsweise Alkali-, Erdalkali-, Ammonium- oder Alkanolaminsalze von Alkylsulfonaten, Alkylsulfaten und Alkylethersulfaten, wie zum Beispiel Natriumlaurylalkoholdiglykolethersulfat, Natrium- oder Triethanolaminsalze von Alkylsulfaten mit 12 bis 18, vorzugsweise 12 bis 14 Kohlenstoffatomen, die Natrium- oder Triethanolaminsalze von Lauryl- oder Tetradecylethersulfaten, das Dinatriumsalz des Sulfosuccinhalbesters von Alkanolamiden, Seifen und Polyethercarbonsäuren;
b) nichtionische oberflächenaktive Agenzien, wie beispielsweise oxethylierte Fettalkohole mit 12 bis 18 Kohlenstoffatomen, zum Beispiel mit bis zu 40 Mol Ethylenoxid pro Mol Fettalkohol oxethylierter Lauryl-, Tetradecyl-, Cetyl- und Stearylalkohol, allein oder im Gemisch, oxethylierte Lanolinalkohole, oxethyliertes Lanolin, oxethylierte Alkylphenole mit 8 bis 30 Kohlenstoffatomen im Alkylrest und 1 bis 10 Ethylenoxideinheiten im Molekül, Fettsäurealkanolamide sowie oxethylierte Sorbitanfettsäureester;
c) kationische oberflächenaktive Agenzien, wie beispielsweise Dilauryldimethylammoniumchlorid, Chloride oder Bromide von Alkyldimethylbenzylammoniumsalzen, Alkyltrimethylammoniumsalze, beispielsweise Cetyltrimethylammoniumchlorid oder -bromid, Tetradecyltrimethylammoniumchlorid oder - bromid, Alkyldimethylhydroxyethylammoniumchloride oder -bromide, Dialkyldimethylammoniumchloride oder -bromide, Alkylpyridiniumsalze, beispielsweise Lauryl- oder Cetylpyridiniumchlorid, Alkylamidoethyltrimethylammoniumethersulfate, Verbindungen mit kationischem Charakter wie Aminoxide, beispielsweise Alkyldimethylaminoxide oder Alkylaminoethyldimethylaminoxide und
d) amphotere oder zwitterionische oberflächenaktive Agenzien wie beispielsweise Carboxylderivate des Imidazols, N-Alkyl- und N-Alkylamidobetaine, N-Alkylsulfobetaine, N-Alkylaminopropionate, Alkyldimethylcarboxymethylammoniumsalze mit 12 bis 18 Kohlenstoffatomen sowie Fettsäurealkylamidobetaine, beispielsweise Fettsäureamidopropyldimethylaminoessigsäurebetain.

Selbstverständlich kann das Fixiermittel alle für derartige Mittel üblichen Zusatzstoffe, zum Beispiel Verdickungsmittel, wie beispielsweise Kaolin, Bentonit, Fettsäuren, höhere Fettalkohole, Stärke, Polyacrylsäure und deren Derivate, Cellulosederivate, Alginate, Vaseline oder Paraffinöl, ferner Farbstoffe, Trübungsmittel, wie zum Beispiel Polyethylenglykolester, oder Alkohole, wie beispielsweise Ethanol, Propanol und Isopropanol, Lösungsvermittler, Puffersubstanzen, Parfümöle, haarkonditionierende oder haarpflegende Bestandteile, wie zum Beispiel Lanolinderivate, Cholesterin, Pantothensäure, Proteinderivate und -hydrolysate, Betain, Provitamine und Vitamine sowie Pflanzenextrakte enthalten.

Die Bestandteile der kosmetischen Zubereitung werden zur Herstellung der gebrauchsfertigen Fixiermittel in für diesen Zweck üblichen Mengen eingesetzt. Beispielsweise werden Netzmittel und Emulgatoren in Konzentrationen von 0,2 bis 30 Gewichtsprozent, Alkohole in einer Konzentration von 1 bis 80 Gew.%, haarkonditionierende oder haarpflegende Bestandteile in einer Konzentration von 0,1 bis 10 Gew.%, und Verdickungsmittel in Konzentrationen von 0,1 bis 25 Gewichtsprozent bezogen auf das gebrauchsfertige Fixiermittel eingesetzt.

Feste Zusätze und mit Dehydroascorbinsäure verträgliche Zusätze können gemeinsam mit der Dehydroascorbinsäure in der Komponente 1 enthalten sein, während die übrigen Zusätze in der flüssigen Komponente 2 enthalten sind.

Die Anwendungstemperatur des gebrauchsfertigen Fixiermittels liegt in einem Bereich von 10°C bis 60°C, vorzugsweise 20°C bis 55°C, besonders bevorzugt 30°C bis 50°C. Die Einwirkungszeiten betragen von 1 bis 45 Minuten, vorzugsweise 3 bis 25 Minuten, besonders bevorzugt 5 bis 15 Minuten.

Das erfindungsgemäße Fixiermittel auf Basis der Dehydoascorbinsäure in der Komponenten 1 ist vorzugsweise wasserfrei. Wasserfrei bedeutet hier nicht, daß jegliche Spuren von Wasser auszuschließen sind. In verwendeten Rohstoffen enthaltenes Kristallwasser oder Feuchtigkeitsspuren sollen lediglich eine Gesamtmenge von maximal 10 Gewichtsprozent, vorzugsweise 4 Gewichtsprozent, besonders bevorzugt 1 Gewichtsprozent, bezogen auf die Komponente 1 nicht überschreiten.

Die Dehydroascorbinsäure, ihr Derivat oder ihr Salz oder deren Gemisch wird in der Komponente 1 alleine oder als Gemisch mit den üblichen, in der Kosmetik eingesetzten Zusatzstoffen in einem wasserfreien Medium, vorzugsweise als staubfreies Pulver, Granulat oder als Tablette verwendet. Beim Einbringen in das geeignete Medium der Komponente 2, welche bevorzugt in eine wäßrige oder wäßrig-alkoholische Lösung darstellt, wird ein gebrauchsfertiges Fixiermittel erhalten, das bevorzugt 1 bis 5 Gewichtsprozent, Dehydroascorbinsäure, ihrer Derivate oder Salze oder deren Gemisch enthält.

Ein weiterer Gegenstand der vorliegenden Erfindung ist ein Verfahren zur dauerhaften Haarverformung, bei dem man das Haar bevor und/oder nachdem man es in die gewünschte Form bringt mit einem keratinreduzierenden Verformungsmittel behandelt, spült, sodann mit einem Fixiermittel oxidativ nachbehandelt, spült, anschließend zur Frisur legt und sodann trocknet, welches dadurch gekennzeichnet ist, daß für die oxidative Nachbehandlung das vorstehend beschriebene Fixiermittel verwendet wird. Die Spülung erfolgt vorzugsweise mit Wasser.

Bei einer besonderen Ausführungsform des erfindungsgemäßen Verfahrens wird das Haar zunächst mit dem keratinreduzierenden Verformungsmittel behandelt, das Verformungsmittel nach der Einwirkungszeit ausgespült, anschließend wird das Haar mit dem vorstehend beschriebenen Fixiermittel auf der Basis von Dehydroascorbinsäure, ihrer Derivate oder Salze als Oxidationsmittel behandelt (vorfixiert) und sodann mit einem Fixiermittel auf der Basis von Wasserstoffperoxid oder Bromat nachbehandelt (nachfixiert). Besonders vorteilhaft weist das Fixiermittel für die Nachfixierung eine geringere Konzentration an Oxidationsmittel auf als für solche Mittel üblich; so beträgt vorzugsweise die Konzentration an Wasserstoffperoxid nur 0,1 bis 1 Gew.% bzw. an Bromat nur 1 bis 5 Gew.%.

Bei dem erfindungsgemäßen Verfahren wird das Haar gewaschen, mit einem Handtuch frottiert, gegebenenfalls mit einem Teil des keratinreduzierenden Verformungsmittels vorgefeuchtet, in einzelne Strähnen aufgeteilt und auf Wickler gewickelt. Der Durchmesser der Wickler beträgt hierbei, je nachdem ob ein Dauerwellung oder eine Entkräuselung der Haare gewünscht wird, entweder etwa 5 bis 13 Millimeter oder etwa 15 bis 35 Millimeter. Auf das gewickelte Haar wird anschließend eine für die dauerhafte Haarverformung ausreichende Menge eines Verformungsmittels aufgetragen. Die für die dauerhafte Haarverformung erforderliche Gesamtmenge des Verformungsmittels beträgt im allgemeinen etwa 80 bis 120 Gramm.

Die bei dem erfindungsgemäßen Verfahren verwendbaren Verformungsmittel enthalten übliche keratinreduzierende Verbindungen, wie zum Beispiel bestimmte Mercaptoverbindungen, insbesondere Thioglykolsäure, Thioglycerin, Cystein, Cysteamin sowie Salze oder Ester von Mercaptocarbonsäuren. Diese Verformungsmittel enthalten die keratinreduzierenden Verbindungen in den für derartige Mittel üblichen Mengen, beispielsweise die Ammoniumsalze der Thioglykol- oder Thiomilchsäure in einer Menge von etwa 2 bis 12 Gewichtsprozent. Der pH-Wert dieser Verformungsmittel beträgt im allgemeinen etwa 7 bis 11, wobei die Einstellung des pH-Wertes vorzugsweise mit Ammoniak, Monoethanolamin, Ammoniumcarbonat oder Ammoniumhydrogencarbonat erfolgt. Bei sauer (zum Beispiel auf pH = 6,5 bis 6,9) eingestellten Verformungsmitteln werden vorzugsweise Ester von Mercaptocarbonsäuren, wie beispielsweise Monothioglykolsäureglykolester oder - glycerinester, in einer Konzentration von etwa 2 bis 25 Gewichtsprozent verwendet.

Die Verformungsmittel können weiterhin alle für derartige Mittel üblichen Zusatzstoffe, beispielsweise Quell- und Penetrationsstoffe, Verdickungsmittel, Netzmittel und Emulgatoren, Alkohole, Lösungsvermittler, Stabilisatoren, Farbstoffe, Parfümöle sowie haarkonditionierende oder haarpflegende Bestandteile, enthalten. Die vorstehend genannten Zusatzstoffe werden in den für solche Zwecke üblichen Mengen verwendet, zum Beispiel die Netzmittel und Emulgatoren in Konzentrationen von etwa 0,2 bis 30 Gewichtsprozent, während die Verdickungsmittel in einer Menge von etwa 0,1 bis 25 Gewichtsprozent in dem Verformungsmittel enthalten sein können.

Das bei dem erfindungsgemäßen Verfahren verwendete Verformungsmittel kann sowohl in Form einer wäßrigen Lösung oder Emulsion als auch in verdickter Form auf wäßriger Basis, insbesondere als Creme, Gel oder Paste, oder in Form eines Aerosolschaums vorliegen.

Nach einer für die dauerhafte Haarverformung ausreichenden Einwirkungszeit, welche je nach der Haarbeschaffenheit, dem pH-Wert und der Verformungswirksamkeit des Verformungsmittels sowie in Abhängigkeit von der Anwendungstemperatur etwa 5 bis 45 Minuten (5 bis 30 Minuten mit Wärmeeinwirkung; 20 bis 45 Minuten ohne Wärmeeinwirkung) beträgt, wird das Haar mit Wasser gespült und sodann mit etwa 50 Gramm bis 350 Gramm, vorzugsweise 80 bis 200 Gramm des vorstehend beschriebenen gebrauchsfertigen Fixiermittels oxidativ nachbehandelt.

Nach einer Einwirkungszeit des Fixiermittels von 1 bis 45 Minuten, vorzugsweise 3 bis 25 Minuten, besonders bevorzugt 5 bis 15 Minuten werden die Wickler entfernt und das abgewickelte Haar, falls erforderlich, nochmals mit dem Fixiermittel oxidativ nachbehandelt. Sodann wird das Haar, vorzugsweise mit Wasser, gespült, zur Frisur gelegt und getrocknet.

Das so behandelte Haar besitzt eine gleichmäßige, nach mehrfachen derartigen Dauerwellbehandlungen zunehmend haltbare Umformung. Im Unterschied zu Haaren, die mit Peroxid fixiert wurden und die eine deutlich feststellbare Farbverschiebung in Richtung rot und gelb aufweisen (Aufhellungsgrad), liegen die Werte bei der erfindungsgemäß verwendeten Fixierung im Bereich unbehandelter Haarsträhnen. Zudem liegt der Cysteinsäuregehalt des so behandelten Haares deutlich unter dem von Haar, das mit einem Fixiermittel auf Basis von Wasserstoffperoxid und Bromat behandelt wurde. Außerdem entsteht bei der Oxidation mit dem hier beschriebenen Fixiermittel kein unangenehmer Mercaptangeruch.

### Vergleichsversuche

### Vergleich der Haarschädigung bei Anwendung von Dehydroascorbinsäure gegenüber Peroxid und Bromat

Die mit dem erfindungsgemäßen Mittel erzielten hervorragenden Resultate werden durch die im folgenden beschriebenen Vergleichsversuche veranschaulicht.

Es werden die Meßwerte (µmol/g) an Cystin und Cysteinsäure sowie der Reißkräfte (Bündelzugfestigkeiten [N]) von 3- und 6-fach behandelten dauergewellten Zählhaarsträhnen, die mit einer frisch zubereiteten Dehydroascorbinsäurelösung, einer gängigen Wasserstoffperoxidfixierung und einer gängigen Bromatfixierung behandelt wurden, verglichen.

Je 2 bis 3 unbehandelte und damit nicht vorgeschädigte Zählhaarsträhnen (bestehend aus je 100 Haaren mit einer Länge von genau 16,5 Zentimetern) aus mitteleuropäischem Haar werden naß auf genormte Spiralwickler (Innendurchmesser: 3 Millimeter) aufgewickelt und nach dem Konditionieren im Klimaraum (Temperatur 20°C; Luftfeuchte: 65%) mit einer handelsübliche Welllösung (Thioglykolsäuregehalt: 10 Gewichtsprozent; pH = 8,2) behandelt. Die Auftragemenge an Wellflüssigkeit wurde über das Verhältnis 1 : 1,2 errechnet (1g Haar: 1,2 ml Wellflüssigkeit). Die Menge von 1,2 ml auf etwa 1 Gramm Haar entspricht einer Menge von 50 ml Wellösung pro Damenkopf mit einem Durchschnittsgewicht von ca. 30g Haar pro Kopf. Als Einwirkungszeit wurden 15 Minuten gewählt; die Einwirkungstemperatur des Reduktionsmittels betrug 50 Grad Celsius. Das Wellmittel auf der Basis von Thioglykolsäure wurde gut mit Wasser ausgespült (2 - 3 Minuten). Anschließend wurden die Zählhaarsträhnen jeweils intensiv mit dem jeweiligen gebrauchsfertigen Fixiermittel befeuchtet. Dieser Vorgang wurde 3 bzw. 6 mal wiederholt, indem die Strähnen nach jeder Behandlung 6 Stunden lang im Wasserbad ausgehängt wurden.

Als Fixiermittel wurden folgende Lösungen verwendet:

2,4%-ige wässrige Lösung auf Basis von Wasserstoffperoxid (Einstellung mit Phosphorsäure auf pH = 2,5), 12%-ige wässrige Natriumbromatlösung (Einstellung mit Natriumphosphat auf pH = 6,4) und eine frisch zubereitete 2,5%-ige wässrige Lösung von Natriumdehydroascorbat (Einstellung mit Natronlauge auf pH = 5,6).

Die Fixierung wurde als Spülfixierung aufgetragen. Die Fixiermittelmenge entspricht ca. 80 bis 100 ml pro 30 Gramm Kopfhaar.

Aussagen über die Vorteile der erfindungsgemäßen Fixierung gegenüber den gängigen Fixiermitteln auf Basis von Wasserstoffperoxid und Bromat ergeben sich aus den Cystin- und Cysteinsäurewerten in µmol/g, welche mittels Aminosäureanalyse bestimmt wurden sowie über die Bestimmung der Bündelzugfestigkeiten. Die Bündelzugfestigkeiten dienen als Maß für die Haarschonung und sind an Einzelhaarsträhnen bestimmt worden.

Für die Einzelhaarreißungen werden dauergewellte Zählhaarsträhnen verwendet. Aus den Strähnen werden je 20 Haare entnommen und mit einer Presse in kleine, vorgefertigte Metallhülsen gequetscht. Anschließend werden die Haare auf ihren Durchmesser hin vermessen und zur Reißung an einem Zug-/Dehnungsmeßgerät mit Probenteller vermessen.

Übersichtstabelle: Mittelwerte der Cystin-, Cysteinsäuregehalte aus 3 Strähen und Bündelzugfestigkeiten aus 20 Einzelhaaren.

| **Fixiermittel** | **Anzahl der Dauerwellbehandlungen** | **Cystin [µmol/g]** | **Cysteinsäure [µmol/g]** | **Bündelzugfestigkeiten [N]** |
|---|---|---|---|---|
| Peroxid | 3 | 541 | 218 | 7,7 |
| Peroxid | 6 | 429 | 321 | 6,9 |
| Bromat | 3 | 541 | 122 | 9,3 |
| Bromat | 6 | 473 | 213 | 7,2 |
| Dehydroascorbinsäure | 3 | 570 | 35 | 8,1 |
| Dehydroascorbinsäure | 6 | 546 | 51 | 8,3 |

Wie aus der Übersichtstabelle hervorgeht, sind die Gehalte an Cystin bei 3-fachdauergewellten Haaren bei allen Fixiermitteln höher als bei 6-fach dauergewellten Haaren. Dies erklärt sich über die mehrfache Reduktion des Haarkeratins. Bei mit Peroxid fixiertem Haar liegen die Cysteinsäuregehalte nach der 3-fach-Behandlung ähnlich hoch wie bei der Bromatfixierung nach der 3-fach Behandlung. Auffallend ist, daß die Cysteinsäurewerte (Maß für die oxidative Haarschädigung) der mit Dehydroascorbinsäure fixierten Haare in etwa dem Blindwert (nicht dauergewellte Strähne: Cysteinsäurgehalt: 41 µmol/g) entsprechen. Infolgedessen tritt bei Verwendung von Dehydroascorbinsäure keine oxidative Schädigung auf. Die Bündelzugfestigkeiten der mit Dehydroascorbinsäure fixierten Strähnen sind nach 3- und 6-fach Behandlungen annähernd gleich, so daß mit Dehydroascorbinsäure ein Oxidationsmittel vorliegt, das auch bei mehrfacher Anwendung keine Zunahme der Haarschädigung aufweist.

Die nachfolgenden Beispiele seien zur genauen Beschreibung des erfindungsgemäßen Verfahrens aufgeführt.

In diesen Beispielen wurde das Haar jeweils in folgender Weise reduktiv vorbehandelt:

Je 3 bis 4 unbehandelte und damit nicht vorgeschädigte Zählhaarsträhnen (bestehend aus je 100 Haaren mit einer Länge von genau 16,5 Zentimetern) aus mittelbraunem mitteleuropäischem Haar wurden naß auf genormte Spiralwickler (Innendurchmesser: 3 Millimeter) aufgewickelt und nach dem Konditionieren im Klimaraum (Temperatur 20°C; Luftfeuchte: 65%) mit einer handelsüblichen Welllösung (Thioglykolsäuregehalt: 10 Gewichtsprozent; pH = 8,2) behandelt. Die Auftragemenge an Wellflüssigkeit wurde über das Verhältnis 1 : 1,2 errechnet (1g Haar: 1,2 ml Wellflüssigkeit). Die Menge von 1,2 ml auf etwa 1 Gramm Haar entspricht einer Menge von 50 ml Wellösung pro Damenkopf mit einem Durchschnittsgewicht von ca. 30g Haar pro Kopf. Als Einwirkungszeit wurden 15 Minuten gewählt; die Einwirkungstemperatur des Reduktionsmittels betrug 50 Grad Celsius.

### Beispiel 1

Nach dem Ende der Einwirkungszeit wird die überschüssige Welllösung mit Wasser ausgespült und sodann die Fixierlösung mit einem Schwamm aufgetragen. Die Fixierlösung wird 2 Minuten vor dem Gebrauch durch Auflösen der Komponente 1 in der Komponente 2 hergestellt.

| Komponente 1 | |
|---|---|
| 5,00 g | bis-Dehydroascorbinsäure, Pulver wasserfrei |

| Komponente 2 | |
|---|---|
| 3,75 g | Natriumlaurylalkoholdiglykolethersulfat |
| 91,25 g | Wasser |

Der pH-Wert der gebrauchsfertigen Fixierung wird mit 25%-iger wäßriger Ammoniaklösung auf 3,5 eingestellt.

Das Haar wird 30 Minuten lang bei 22 Grad Celsius fixiert und anschließend mit Wasser gespült. Sodann werden die Wickler entfernt und das Haar mit lauwarmem Wasser ausgespült. Schließlich wird das Haar zur Frisur gelegt und sodann getrocknet.

Das so behandelte Haar zeigt einen guten Allgemeinzustand, ist nicht aufgehellt und frei von störendem Mercaptangeruch.

### Beispiel 2

Analog zu Beispiel 1 werden die Haare mit einer 1 Minute vor dem Gebrauch aus den nachstehenden Komponenten 1 und 2 hergestellten Fixierlösung behandelt.

| Komponente 1 | |
|---|---|
| 5,0 g | Dehydroascorbinsäure, Pulver wasserfrei |

| Komponente 2 | |
|---|---|
| 0,5 g | ethoxyliertes Fettalkoholsulfat |
| 0,2 g | Parfümöl |
| | Citratpuffer bis pH = 6,0 |
| ad 100,00g | Wasser |

Das so behandelte Haar zeigt eine gute Umformung und Haltbarkeit, ist nicht aufgehellt und frei von störendem Mercaptangeruch.

### Beispiel 3

Die Haare werden mit einer 3 Minuten vor dem Gebrauch aus den nachfogenden Komponenten 1 und 2 hergestellten Fixierlösung analog Beispiel 1 behandelt:

| Komponente 1 | |
|---|---|
| 2,5 g | Dehydroascorbinsäure, Pulver wasserfrei |

| Komponente 2 | |
|---|---|
| 1,0 g | Dimethylaminoethylmethacrylat, zu 75 % mit Dimethylsulfat quaternisiert |
| 0,6 g | Fettsäureamidopropyl-dimethylaminoessigsäurebetain |
| 0,5 g | Lauryldimethylaminoxid |
| 0,5 g | Citratpuffer |
| | Natriumhydroxid bis pH 5,6 |
| ad 100,00g | Wasser |

Das so behandelte Haar ist in gutem Zustand, nicht aufgehellt und frei von störendem Mercaptangeruch.

### Beispiel 4

Im Anschluß an eine Behandlung der Haare mit dem Verformungsmittel auf Thioglykolsäurebasis wird das Haar mit Wasser gespült und eine aus den nachfolgenden Komponenten 1 und 2 kurz vor der Anwendung hergestelltes gebrauchsfertiges Fixiermittel mit einem Schwamm aufgetragen.

| **Komponente 1** | |
|---|---|
| 3,0 g | bis-Dehydroascorbinsäure als Tablette |

| **Komponente 2** | |
|---|---|
| 0,30 g | Rizinusöl, oxethyliert mit 40 Mol Ethylenoxid |
| 0,30 g | Octylphenol, oxethyliert mit 20 Mol Ethylenoxid |
| 0,10 g | alpha-Bisabolol |
| ad 100 g | Wasser |

Die Komponenten 1 und 2 werden 5 Minuten vor dem Gebrauch miteinander verrührt und das so erhaltene Gemisch mit 4 Liter Wasser verdünnt.

Im Anschluß an eine Behandlung der Haare mit dem Verformungsmittel auf Thioglykolsäurebasis wird das Haar mit Wasser gespült. Sodann wird das Haar mit dem vorstehend beschriebenen gebrauchsfertigen Fixiermittel unter Verwendung einer Umpumpvorrichtung 5 Minuten lang gespült. Nach dem Entfernen der Wickler wird das Haar mit Wasser gespült, zur Frisur gelegt und danach getrocknet. Es wird ein gut gewelltes Haar erhalten.

### Beispiel 5

Die Reduktion der Haare wird wie eingangs beschrieben vorgenommen. Nach dem Ausspülen mit Wasser wird das Haar wie folgt fixiert:

Die folgende Zusammensetzung ist als "Vor- und Schnellfixierung" in Kombination mit einem Fixiermitteln auf Wasserstoffperoxid-Basis geeignet:

### Vorfixiermittel

| Komponente 1 | |
|---|---|
| 1,50 g | Dehydroascorbinsäure, Pulver |

| Komponente 2 | |
|---|---|
| 1,00 g | Nonoylphenolpolyglykolether |
| 0,10 g | kationisches Polymer (CTFA: Polyquarternium-10) |
| 0,20 g | Parfüm |
| | Citratpuffer zur pH-Einstellung auf 5,0 |
| ad 100g | Wasser |

Die Komponente 1 wird 30 Sekunden vor dem Gebrauch in der auf 50 Grad Celsius erwärmten Komponente 2 aufgelöst.

### Die Endfixierung erfolgt mit folgender Zusammensetzung:

| | |
|---|---|
| 0,90g | Wasserstoffperoxid |
| 0,02g | Phenacetin |
| 0,10g | Parfüm |
| 2,00g | Natriumlaurylethersulfat |
| | Phosphorsäure zur pH-Einstellung auf 3,5 |
| ad 100g | Wasser |

Im Anschluß an eine Behandlung der Haare mit dem Verformungsmittel auf Thioglykolsäurebasis wird das Haar mit Wasser gespült und sodann mit dem vorstehend beschriebenen Vorfixiermittel 10 Minuten lang behandelt (Schwammauftragung). Anschließend wird die Endfixierung aufgetragen und 5 Minuten lang einwirken gelassen. Nach der Entfernung der Wickler wird das Haar mit Wasser gespült, zur Frisur gelegt und getrocknet.

Das so behandelte Haar ist gut gelockt, nicht aufgehellt und frei von störendem Mercaptangeruch.

### Beispiel 6

| Komponente 1 | |
|---|---|
| 4,0 g | bis-Dehydroascorbinsäure, Pulver wasserfrei |

| Komponente 2 | |
|---|---|
| 0,1 g | Parfümöl |
| 96,0 g | Wasser |

Komponente 1 wird 20 Sekunden vor dem Gebrauch in Komponente 2 gelöst. Der pH-Wert der gebrauchsfertigen Zubereitung beträgt 2,2. Die Temperatur der gebrauchsfertigen Zubereitung wurde auf 45°C eingestellt.

Im Anschluß an eine Behandlung der Haare mit dem Verformungsmittel auf Thioglykolsäurebasis wird das Haar mit Wasser gespült und sodann mit dem vorstehend beschriebenen Fixiermittel unter Verwendung einer Umpumpvorrichtung mit Temperaturkontrolle bei 45°C 10 Minuten lang gespült. Nach Entfernung der Wickler wird das Haar mit Wasser gespült, zur Frisur gelegt und getrocknet.

Das so behandelte Haar ist nicht aufgehellt und frei von störendem Mercaptangeruch.

## Patentansprüche

1. Mittel zur Durchführung der oxidativen Nachbehandlung bei der dauerhaften Haarverformung mit einem Gehalt an Dehydroascorbinsäure, **dadurch gekennzeichnet, dass** es 1 bis 10 Gew.% Dehydroascorbinsäure ihre Derivate oder Salze oder deren Gemisch enthält und erhalten wird durch Vermischen von 2 Komponenten unmittelbar (10 sec bis 20 min) vor dem Gebrauch, wobei die Komponente 1 Dehydroascorbinsäure, ihre Derivate oder Salze oder deren Gemisch, wasserfrei oder mit bis zu 10 Gewichtsprozent Wasser, als Pulver-, Granulat- oder Tablette oder mikroverkapselt oder als Suspension enthält und die Komponente 2 als wäßrige, alkoholische oder wäßrig-alkoholische Zubereitung vorliegt.

2. Verwendung von Dehydroascorbinsäure, ihrer Derivate oder Salze oder deren Gemisch zur Durchführung der oxidativen Nachbehandlung bei der dauerhaften Haarverformung, **dadurch gekennzeichnet, dass** man die Dehydroascorbinsäure, ihre Derivate oder Salze in einer Menge von 1 bis 10 Gew.% in einem gebrauchsfertigen Fixiermittel verwendet, das unmittelbar (10 sec bis 20 min) vor dem Gebrauch durch Vermischen von 2 Komponenten erhalten wird, wobei die Komponente 1 die Dehydroascorbinsäure, ihre Derivate oder Salze oder deren Gemisch, wasserfrei oder mit bis zu 10 Gewichtsprozent Wasser, als Pulver-, Granulat- oder Tablette oder mikroverkapselt oder als Suspension enthält und die Komponente 2 als wäßrige, alkoholische oder wäßrig-alkoholische Zubereitung vorliegt.

3. Verfahren zur dauerhaften Haarverformung, bei dem man das Haar bevor und/oder nachdem man es in die gewünschte Form bringt, mit einem keratinreduzierenden Verformungsmittel behandelt, danach spült, sodann mit einem Fixiermittel oxidativ nachbehandelt und erneut spült, **dadurch gekennzeichnet, dass** man ein Fixiermittel verwendet, das 1 bis 10 Gew.% Dehydroascorbinsäure enthält und unmittelbar (10 sec bis 20 min) vor dem Gebrauch durch Vermischen von 2 Komponenten erhalten wird, wobei die Komponente 1 die Dehydroascorbinsäure, ihre Derivate oder Salze oder deren Gemisch, wasserfrei oder mit bis zu 10 Gewichtsprozent Wasser, als Pulver-, Granulat- oder Tablette oder mikroverkapselt oder als Suspension enthält und die Komponente 2 als wäßrige, alkoholische oder wäßrig-alkoholische Zubereitung vorliegt.

4. Verfahren nach Anspruch 3, **dadurch gekennzeichnet, dass** man das Haar zunächst mit dem keratinreduzierenden Verformungsmittel behandelt, das Verformungsmittel nach der Einwirkungszeit ausgespült, anschließend das Haar mit einem in Anspruch 3 definierten Fixiermittel auf der Basis von Dehydroascorbinsäure, ihrer Derivate oder Salze als Oxidationsmittel behandelt (vorfixiert) und sodann mit einem Fixiermittel auf der Basis von Wasserstoffperoxid oder Bromat nachbehandelt (nachfixiert).

## Claims

1. Agent for carrying out the oxidative after-treatment during permanent hair shaping with a content of dehydroascorbic acid, **characterized in that** it comprises 1 to 10% by weight of dehydroascorbic acid, its derivatives or salts or mixture thereof and is obtained by mixing two components directly (10 sec to 20 min) prior to use, where component 1 comprises dehydroascorbic acid, its derivatives or salts or mixture thereof, anhydrous or with up to 10% by weight of water, as powder, granules or tablet or microencapsulated or as suspension, and component 2 is in the form of an aqueous, alcoholic or aqueous-alcoholic preparation.

2. Use of dehydroascorbic acid, its derivatives or salts or mixture thereof for carrying out the oxidative after-treatment during permanent hair shaping, **characterized in that** the dehydroascorbic acid, its derivatives or salts are used in an amount of from 1 to 10% by weight in a ready-to-use neutralizer which is obtained directly (10 sec to 20 min) prior to use by mixing two components, where component 1 comprises the dehydroascorbic acid, its derivatives or salts or mixture thereof, anhydrous or with up to 10% by weight of water, as powder, granules or tablet or microencapsulated or as suspension, and component 2 is in the form of an aqueous, alcoholic or aqueous-alcoholic preparation.

3. Method of permanent hair shaping in which the hair, before and/or after it is shaped as desired, is treated with a keratin-reducing shaping agent, then rinsed, then oxidatively after-treated with a neutralizer and rinsed again, **characterized in that** a neutralizer is used which comprises 1 to 10% by weight of dehydroascorbic acid and is obtained directly (10 sec to 20 min) prior to use by mixing two components, where component 1 comprises the dehydroascorbic acid, its derivatives or salts or mixture thereof, anhydrous or with up to 10% by weight of water, as powder, granules or tablet or microencapsulated or as suspension, and component 2 is in the form of an aqueous, alcoholic or aqueous-alcoholic preparation.

4. Method according to Claim 3, **characterized in that** the hair is firstly treated with a keratin-reducing shaping agent, the shaping agent is rinsed out after the contact time, then the hair is treated (preneutralized) with a neutralizer defined in Claim 3 and based on dehydroascorbic acid, its derivatives or salts as oxidizing agent, and then after-treated (after-neutralized) with a neutralizer based on hydrogen peroxide or bromate.

## Revendications

1. Composition pour l'exécution de l'après-traitement oxydant dans la mise en forme permanente des cheveux, ayant une teneur en acide déshydroascorbique, **caractérisée en ce qu'**elle contient de 1 à 10 % en poids d'acide déshydroascorbique, de ses dérivés ou sels ou d'un mélange de ceux-ci et est obtenue par mélange de deux composants immédiatement (10 secondes à 20 minutes) avant l'emploi, le composant 1 contenant de l'acide déshydroascorbique, ses dérivés ou sels ou un mélange de ceux-ci, anhydre(s) ou comportant jusqu'à 10 % en poids d'eau, sous forme de poudre, de produit granulé ou de comprimé ou microencapsulé(s) ou sous forme de suspension et le composant 2 se trouvant sous forme de préparation aqueuse, alcoolique ou aqueuse-alcoolique.

2. Utilisation d'acide déshydroascorbique, de ses dérivés ou sels ou d'un mélange de ceux-ci, pour l'exécution de l'après-traitement oxydant dans la mise en forme permanente des cheveux, **caractérisée en ce qu'**on utilise l'acide déshydroascorbique, ses dérivés ou sels en une quantité de 1 à 10 % en poids dans un fixateur prêt à l'emploi qui est obtenu immédiatement (10 secondes à 20 minutes) avant l'emploi, par mélange de deux composants, le composant 1 contenant l'acide déshydroascorbique, ses dérivés ou sels ou un mélange de ceux-ci, anhydre(s) ou comportant jusqu'à 10 % en poids d'eau, sous forme de poudre, de produit granulé ou de comprimé ou microencapsulé(s) ou sous forme de suspension et le composant 2 se trouvant sous forme de préparation aqueuse, alcoolique ou aqueuse-alcoolique.

3. Procédé pour la mise en forme permanente des cheveux, dans lequel on traite les cheveux, avant et/ou après leur mise en la forme désirée, par un agent de déformation réduisant la kératine, puis on les rince, ensuite on les soumet à un après-traitement oxydant par un fixateur et on les rince à nouveau, **caractérisée en ce qu'**on utilise un fixateur qui contient de 1 à 10 % en poids d'acide déshydroascorbique et est obtenu immédiatement (10 secondes à 20 minutes) avant l'emploi, par mélange de deux composants, le composant 1 contenant l'acide déshydroascorbique, ses dérivés ou sels ou un mélange de ceux-ci, anhydre(s) ou comportant jusqu'à 10 % en poids d'eau, sous forme de poudre, de produit granulé ou de comprimé ou microencapsulé(s) ou sous forme de suspension et le composant 2 se trouvant sous forme de préparation aqueuse, alcoolique ou aqueuse-alcoolique.

4. Procédé selon la revendication 3, **caractérisé en ce qu'**on traite d'abord les cheveux par l'agent de déformation réduisant la kératine, on élimine par rinçage l'agent de déformation après le temps d'action, puis on traite (préfixe) les cheveux par un fixateur défini dans la revendication 3, à base d'acide déshydroascorbique, de ses dérivés ou sels en tant qu'oxydant et ensuite on les soumet à un après-traitement (après-fixage) par un fixateur à base de peroxyde d'hydrogène ou de bromate.
